# EUROPEAN PATENT APPLICATION

(11) **EP 3 865 110 A1**
(43) Date of publication of application: **18.08.2021**
(21) Application number: 21155987.7
(22) Date of filing: 09.02.2021
(51) Int. Cl.: A61K 6/17, A61K 6/62, A61K 6/76, A61K 6/77, A61K 6/887

(54) **DENTAL RESTORATION COMPOSITE RESIN**

(30) Priority: 12.02.2020 JP 2020021713
(71) Applicant: GC Corporation, Sunto-gun, Shizuoka 410-1307 (JP)
(72) Inventor: MORI, Toshiki, Itabashi-ku,, Tokyo 174-8585 (JP); KATO, Hiroki, Itabashi-ku,, Tokyo 174-8585 (JP)
(74) Representative: Müller-Boré & Partner Patentanwälte PartG mbB

(57) **Abstract**

A dental restoration composite resin contains a polymerizable monomer, a chain transfer agent, and inorganic particles, wherein the inorganic particles contain inorganic particles (A) having a volume-median particle size of 0.1 µm or more and 0.9 µm or less, the inorganic particles (A) being surface-treated with compounds represented by the general formula (1), the general formula (2), or both.

## Description

This application is based upon and claims priority to Japanese Patent Application No. 2020-021713, filed on February 12, 2020, the entire contents of which are incorporated herein by reference.

The present invention relates to a dental restoration composite resin.

Conventionally, dental restoration composite resins (composite resins for dental filling) are known as restoration materials that directly fill cavities in teeth cavities.

When dental restoration composite resins are used to restore teeth, the dental restoration composite resins shrink as the composite resins cure. As a result, shrinkage stress occurs at the interface between fovea walls and the dental restoration composite resins. When high shrinkage stress increases, the risks of secondary caries due to the cracks in the teeth surfaces and peeling off of cured dental restoration composite resins increase.

In contrast, dental restoration composite resins require mechanical strength to withstand occlusal pressure that may be exerted on teeth.

Patent Document 1 discloses a photopolymerizable dental composition containing (A) a radical polymerizable monomer, (B) an α-diketone compound, (C) an amine compound, (D) a photoacid generator, and (E) an α-alkyl styrene compound.

Patent Document 1: Japanese Patent Application Laid-Open No. 2016-169180

However, the mechanical strength of the cured product of the photopolymerizable dental composition according to Patent Document 1 is insufficient.

One aspect of the invention is to provide a dental restoration composite resin with low shrinkage stress upon curing and high mechanical strength of a cured product.

In one aspect of the invention, a dental restoration composite resin contains a polymerizable monomer, a chain transfer agent, and inorganic particles, wherein the inorganic particles contain inorganic particles (A) having a volume-median particle size of 0.1 µm or more and 0.9 µm or less, the inorganic particles (A) being surface-treated with compounds represented by the following general formula (1), the following general formula (2), or both.

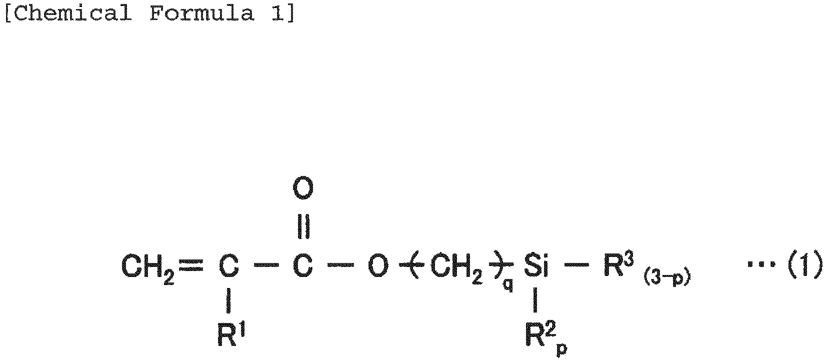

In the formula, R¹ is a hydrogen atom or a methyl group, R² is a hydrolyzable group, R³ is a hydrocarbon group of 1 to 6 carbon atoms, p is 2 or 3, and q is an integer of 8 to 13.

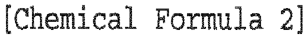

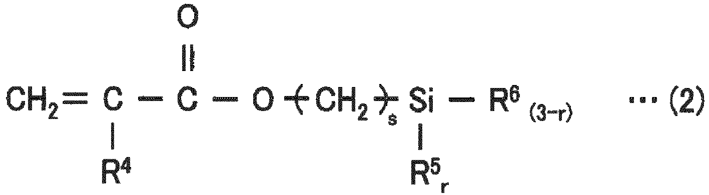

In the formula, R⁴ is a hydrogen atom or a methyl group, R⁵ is a hydrolyzable group, R⁶ is a hydrocarbon group of 1 to 5 carbons, r is 2 or 3, and s is an integer of 1 to 7.

According to one aspect of the invention, the invention is to provide a dental restoration composite resin with low shrinkage stress upon curing and high mechanical strength of a cured product.

Embodiments for carrying out the present invention will be described hereinafter.

### [Dental Restoration Composite Resin]

A dental restoration composite resin of the present embodiment contains a polymerizable monomer, a chain transfer agent, and inorganic particles.

Here, when the dental restoration composite resin is chemically polymerizable, photopolymerizable, or both, a first agent containing an oxidizing agent and a second agent containing a reducing agent are separately packaged as part of a two-component system. The two-components of the dental restoration composite resin are mixed immediately before use.

In contrast, when the dental restoration composite resin is photopolymerizable but not chemically polymerizable, the dental restoration composite resin is a single-component dental restoration composite resin.

The dental restoration composite resin of the present embodiment includes, for example, a paste and the like.

An extrusion strength of the dental restoration composite resin of the present embodiment is typically 10 kgf or less.

When the dental restoration composite resin of the present embodiment is a single-component, the dental restoration composition resin may be packed in a syringe filled with the dental restoration composite resin. A plunger fitted into the syringe from the rear end of the syringe and a needle tip mounted to the tip of the syringe are used.

An inner diameter of a needle is typically 0.3 to 0.9 mm.

When the dental restoration composite resin of the present embodiment is a two-components, the dental restoration composite resin may be packaged in, for example, two syringes that are connected in parallel, and a static mixer may be provided near the tip of the two syringes.

### [Polymerizable Monomer]

A refractive index of a polymer of a polymerizable monomer is 1.52 to 1.58, and preferably 1.53 to 1.58.

The refractive index indicates the refractive index at 25°C as measured by an Abbe refractometer.

The polymerizable monomer is preferably a radical polymerizable monomer and even more preferably (meth)acrylate.

In the scope of the present specification and claims, the term "(meth)acrylate" may refer to an acrylate or a methacrylate, and the (meth)acrylate has one or more of a (meth)acryloyloxy group. In addition, the term "(meth)acryloyloxy group" may refer to a methacryloyloxy group, an acryloyloxy group, or both.

Examples of radical polymerizable monomers include α-cyanoacrylic acid ester, (meth)acrylic acid ester, α-haloacrylic acid ester, crotonic acid ester, cinnamic acid ester, sorbic acid ester, maleic acid ester, itaconic acid ester, (meth)acrylamide, (meth)acrylamide derivative, vinyl ester, vinyl ether, mono-N-vinyl derivative, styrene derivative, and the like. The above radical polymerizable monomers may be used in combination of two or more. Among these, the (meth)acrylate ester and (meth)acrylamide derivative are preferably used, and (meth)acrylate ester is more preferably used.

Examples of monofunctional radical polymerizable monomers include methyl (meth)acrylate, isobutyl (meth)acrylate, benzyl (meth)acrylate, lauryl (meth)acrylate, 2,3-dibromopropyl (meth)acrylate, 2-hydroxyethyl (meth)acrylate, 6-hydroxyhexyl (meth)acrylate, 10-hydroxydecyl (meth)acrylate, propylene glycol mono (meth)acrylate, glycerin mono (meth)acrylate, erythritol mono (meth)acrylate, N-methylol (meth)acrylamide, N-hydroxyethyl (meth)acrylamide, N-dihydroxyethyl (meth)acrylamide, (meth)acryloyloxide decylpyridinium chloride, (meth)acryloyloxy hexadecylpyridinium chloride, (meth)acryloyloxy decylammonium chloride, and the like.

Examples of bifunctional radical polymerizable monomers include ethylene glycol di(meth)acrylate, triethylene glycol di(meth)acrylate, propylene glycol di(meth)acrylate, neopentyl glycol di(meth)acrylate, 1,6-hexanediol di(meth)acrylate, 1,10-decanediol di(meth)acrylate, 2,2-bis[4-[3-(meth)acryloyloxy-2-hydroxypropoxy]phenyl]propane, 2,2-bis[4-(2-(meth)acryloyloxyethoxy)phenyl]propane, 2,2-bis[4-(meth)acryloyloxypolyethoxy]propane,1,2-bis(3-(meth)acryloyloxy-2-hydroxypropoxy)ethane, pentaerythritol di(meth)acrylate, [2,2,4-trimethylhexamethylene bis(2-carbamoyloxyethyl)]di(meth)acrylate, and the like.

Examples of trifunctional radical polymerizable monomers include trimethylolpropane tri(meth)acrylate, trimethylolethane tri(meth)acrylate, tetramethylolmethane tri(meth)acrylate, pentaerythritol tetra(meth)acrylate, dipentaerythritol hexa(meth)acrylate, N,N'-(2,2,4-trimethylhexamethylene)bis[2-(aminocarboxy)propane-1,3-diol]tetramethacrylate, 1,7-diacryloyloxy-2,2,6,6-tetraacryloyloxymethyl-4-oxyheptane, and the like.

### [Chain Transfer Agent]

A chain transfer agent may be a monoolefin, mercaptan, terpenoid, dithiobenzoate, dithiocarbamate, trithiocarbonate, xanthate, and the like that substituted by a phenyl group.

Examples of monoolefins substituted by phenyl groups include 2-phenyl-1-propene (a-methylstyrene), 2-phenyl-1-butene, 2,4-diphenyl-4-methyl-1-pentene (a-methylstyrene dimer), 3,5-diphenyl-5-methyl-2-heptene, 2,4,6-triphenyl-4,6-dimethyl-1-heptene, 3,5,7-triphenyl-5-ethyl-7-methyl-2-nonene, 1,3-diphenyl-1-butene, 2,4-diphenyl-4-methyl-2-pentene, 3,5-diphenyl-5-methyl-3-heptene, 1,1-diphenylene, 2,4-diphenyl-4-methyl-1-pentene, 2-phenyl-1-propene, 1,3-diphenyl-1-butene, and the like.

Examples of mercaptans include 1-butanethiol, 1-octanethiol, 1-octadecanethiol, thiophenol, cyclohexyl 3-mercaptopropionate, 1-decanthiol, 1-dodecanthiol, hexyl 3-mercaptopropionate, 2-mercaptoethanol, 3-mercapto-1,2-propanediol, mercaptoacetic acid, sodium 2-mercaptoethanesulfonate, 3-mercaptopropionic acid, methyl mercaptoacetate, mercaptosuccinic acid, methyl 3-mercaptopropionate, octyl 3-mercaptopropionate, tridecyl 3-mercaptopropionate, octadecyl 3-mercaptopropionate, and the like.

Examples of terpenoids include γ-terpinene, limonene, myrcene, α-terpinene, β-terpinene, terpinolene, β-pinene, and the like.

Examples of dithiobenzoate include cumyl α-pinene dithiobenzoate, tetramethylthiuram disulfide, tetraethylthiuram disulfide, benzodithioic acid, S-(thiobenzoyl)thioglycolic acid, and the like.

Examples of dithiocarbamates include cyanomethyl methyl(phenyl)carbamodithioate and the like.

Examples of trithiocarbonates include 4-cyano-4-[[(dodecylthio)carbonothioyl]thio]pentanoic acid, 2-(dodecylthiocarbonothioylthio)-2-methylpropionic acid, 2-(dodecylthiocarbonothioylthio)-2-methylpropionate N-hydroxysuccinimidyl, and the like.

Examples of xanthate include DITHIOCARBONIC ACID S-TERT-BUTYL ESTER O-ETHYL ESTER, potassium ethylxanthate, and the like.

Among these, α-methylstyrene dimer, 1-decanthiol, γ-terpinene, or cumyl dithiobenzoate are preferably used from the viewpoint of low shrinkage stress upon curing. An α-methylstyrene dimer is more preferably used because the α-methylstyrene dimer is colorless and has no odor.

The chain transfer agent may be used alone or in combination of two or more.

The mass ratio of the chain transfer agent with respect to the polymerizable monomer in the dental restoration composite resins of the present embodiment is preferably 0.1% to 5%, and further preferably 0.5% to 1%. When the mass ratio of the chain transfer agent with respect to the polymerizable monomer in the dental restoration composite resin according to the present embodiment is 0.1% or more, the shrinkage stress upon curing of the dental restoration composite resin according to the present embodiment is further reduced. When the mass ratio is 5% or less, the mechanical strength of the dental composite resin according to the present embodiment improves.

### [Inorganic Particles]

Inorganic particles include inorganic particles (A) and further preferably include inorganic particles (B).

The inorganic particles contain inorganic particles (A) having a volume-median particle size of 0.1 µm or more and 0.9 µm or less, the inorganic particles (A) being surface-treated with compounds represented by the following general formula (1), the following general formula (2), or both.

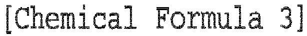

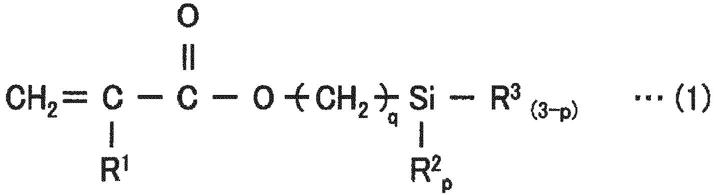

In the formula, R¹ is a hydrogen atom or a methyl group, R² is a hydrolyzable group, R³ is a hydrocarbon group of 1 to 6 carbon atoms, p is 2 or 3, and q is an integer of 8 to 13.

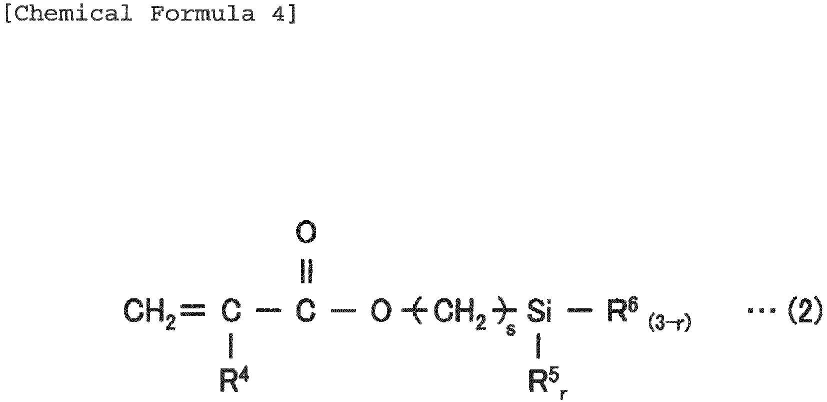

In the formula, R⁴ is a hydrogen atom or a methyl group, R⁵ is a hydrolyzable group, R⁶ is a hydrocarbon group of 1 to 5 carbons, r is 2 or 3, and s is an integer of 1 to 7.

The mass ratio of inorganic particles (A) with respect to polymerizable monomers in the dental restoration composite resins of the present embodiment is preferably 100 to 400% and more preferably 150 to 350%. When the mass ratio of the inorganic particle (A) with respect to the polymerizable monomer in the dental restoration composite resin of the present embodiment is 150% or more, the mechanical strength of the dental restoration composite resin of the present embodiment improves. When the mass ratio is 350% or less, the handleability of the dental restoration composite resin of the present embodiment improves.

The inorganic particles further preferably contain inorganic particles (B) having average primary particle size of 5 nm or more and 50 nm or less, the inorganic particles (B) have groups represented by the following general formula (3), the following general (4), or both at the surfaces of inorganic particles.

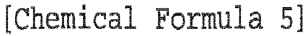

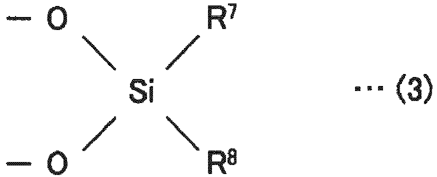

In the formula, R⁷ and R⁸ are independently a methyl group or an ethyl group.

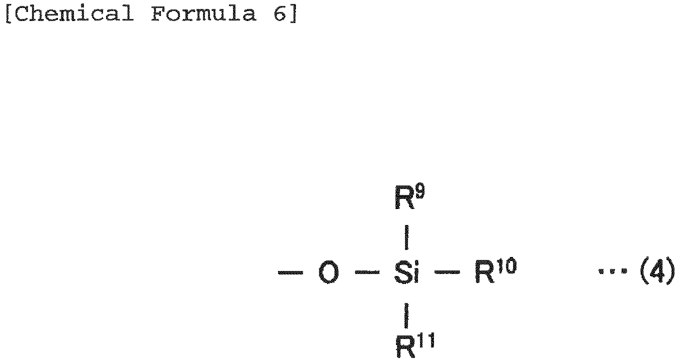

In the formula, R⁹, R¹⁰, and R₁₁ are independently a methyl group or an ethyl group.

### [Inorganic Particles (A)]

Inorganic particles (A) are surface-treated with a compound represented by the general formula (1), a compound represented by the general formula (2), or both. However, when the inorganic particles (A) are surface-treated with a compound represented by the general formula (1) and a compound represented by the general formula (2), the mass ratio of the compound represented by the general formula (1) with respect to the compound represented by the general formula (2) is preferably 1 or more and more preferably 3 or more. When the mass ratio of the compound represented by the general formula (1) with respect to the compound represented by the general formula (2) is 1 or more, the handleability and the mechanical strength of a cured product of the dental restoration composite resin in the present embodiment improves.

A volume-median particle size of the inorganic particles (A) is preferably 0.1 to 0.9 µm and more preferably 0.15 to 0.70 µm. When the volume-median particle size of the inorganic particles (A) is 0.1 µm or more, the handleability of the dental restoration composite resin of the present embodiment improves. When the volume-median particle size of the inorganic particles (A) is 0.9 µm or less, the mechanical strength of the cured product of the dental restoration composite resin of the present embodiment improves.

The volume-median particle size of the inorganic particles (A) can be measured by a laser diffraction-scattering method.

The inorganic particles (A) may be spherical but are preferably irregularly-shaped particles. By having an irregularly-shaped inorganic particle, a specific surface area of the inorganic particles (A) increases. Thereby, a binding ability to a polymerizable monomer increases, and the mechanical strength of the cured product of the dental restoration composite resin of the present embodiment improves.

R² in the general formula (1) and R⁵ in the general formula (2) are not particularly limited as long as the groups are hydrolyzable. Examples of R² in the general formula (1) and R⁵ in the general formula (2) include alkoxy groups such as a methoxy group, ethoxy group, butoxy group, chlorine atoms, isocyanate group, and the like.

R³ in the general formula (1) and R⁶ in the general formula (2) include, for example, alkyl groups having 1 to 6 carbon atoms, alkenyl groups having 2 to 6 carbon atoms, alkynyl groups having 2 to 6 carbon atoms, and the like.

Alkyl groups having 1 to 6 carbon atoms, alkenyl groups having 2 to 6 carbon atoms, and alkynyl groups having 2 to 6 carbon atoms may be linear, branched, or cyclic.

Examples of the compounds represented by the general formula (1) include 8-methacryloyloxyoctyl trimethoxysilane, 9-methacryloyloxynonyl trimethoxysilane, 10-methacryloyloxy decyltrimethoxysilane, 11-methacryloyloxy undecyltrimethoxysilane, 11-methacryloyloxy undecyldichloromethylsilane, 11-methacryloyloxy undecyltrichlorosilane, 11-methacryloyloxy undecyloxyundecylcyclosilane, 11-methacryloyloxy undecyldimethoxymethylsilane, 12-methacryloyloxy dodecyltrimethoxysilane, 13-methacryloyloxy tridecyltrimethoxysilane, and the like. The above compounds may be used in combination of two or more.

Examples of the compounds represented by the general formula (2) include 3-methacryloyloxy propyltrimethoxysilane, 3-acryloyloxy propyltrimethoxysilane, 3-methacryloyloxy propyltriethoxysilane, 3-methacryloyloxy propyldimethoxysilane, 4-methacryloyloxy butyltrimethoxysilane, and the like. The above compounds may be used in combination of two or more.

A surface treatment method using the compound represented by the general formula (1), the compound represented by the general formula (2), or both is not particularly limited. Examples of the surface treatment methods include spraying a solution in which the compound represented by the general formula (1), the compound represented by the general formula (2), or both may be diluted with a solvent while stirring the inorganic particles (A) prior to being surface-treated in a mixing vessel, and heating and drying the particles in the vessel for a certain period of time while continuing to stir; mixing the inorganic particles (A) prior to being surface-treated with the compound represented by the general formula (1), the compound represented by the general formula (2), or both by heating and drying the particles in a solvent; and the like.

A mass ratio of the group derived from the compound represented by the general formula (1), the group derived from the compound represented by the general formula (2), or both with respect to the polymerizable monomer in the dental restoration composite resin of the present embodiment is 0.25% to 7.5% and preferably 0.5 to 6.5%.

A mass ratio of the compound represented by the general formula (1), the compound represented by the general formula (2), or both with respect to the inorganic particles (A) prior to being surface-treated is 0.005 to 0.15 and preferably 0.01 to 0.13.

A refractive index of the inorganic particles (A) is 1.52 to 1.58 and preferably 1.53 to 1.58.

The difference between the refractive index of the polymer of polymerizable monomer and the inorganic particles (A) is 0.03 or less.

Examples of the materials constituting the inorganic particles (A) prior to being surface-treated include silica. As needed-based, various kinds of glass such as an oxide of heavy metal, boron, aluminum, or the like (such as E glass, barium glass, and lanthanum glass-ceramics); various kinds of ceramics; composite oxides (such as silica-titania composite oxide and silica-zirconia composite oxide); kaolin; clay minerals (such as montmorillonite); mica; ytterbium fluoride; yttrium fluoride; and the like can be used. The above materials may be used in combination of two or more.

Commercially available products of the inorganic particles (A) include G018-053, GM27884, 8235, and GM31684 (all of which are manufactured by Schott AG), and E2000 and E3000 (both of which are manufactured by ESSTECH, Inc.).

### [Inorganic Particle (B)]

The dental restoration composite resin further preferably contains inorganic particles (B) in the inorganic particles. In particular, the inorganic particles further include inorganic particles (B) having the groups represented by the general formula (3), the groups represented by the general formula (4), or both, at surfaces of the inorganic particles (B).

The average primary particle size of the inorganic particles (B) is 5 to 50 nm and preferably 5 to 20 nm. When the average primary particle size of the inorganic particles (B) is 5 nm or more, the handleability of the dental restoration composite resin of the present embodiment improves. When the average primary particle size of the inorganic particles (B) is 50 nm or less, the mechanical strength of the dental restoration composite resin of the present embodiment improves.

The average primary particle size of the inorganic particles (B) is the average value of the primary particle diameters of 100 inorganic particles (B) randomly selected after taking on the electron micrographs.

The inorganic particles (B) may be either spherical or irregularly-shaped. Furthermore, the inorganic particles (B) may be either primary particles not aggregated or secondary particles in which primary particles aggregate.

If the primary particles of the inorganic particles (B) have an irregular shape, the primary particle size is the average value of the long diameter and the short diameter of the primary particles.

Surface-treatment methods of the inorganic particles (B) are not limited in.particular. Examples of the methods include spraying the inorganic particles (B), prior to being surface-treated, with a solution of a silane coupling agent diluted with a solvent while being stirred in a mixing tank and are heated and dried for a certain time in the tank while being kept stirred; and stirring and mixing the inorganic particles (A), prior to being surface-treated, with a silane coupling agent in a solvent and heating the mixture thereafter to be dried.

Silane coupling agents are not limited in particular as long as the silane coupling agents can introduce the group represented by the general formula (3), the group represented by the general formula (4), or both onto surfaces. Examples of silane coupling agents include dimethyldichlorosilane, hexamethyldisilazane, and the like.

Materials constituting the inorganic particles (B) prior to being surface-treated include inorganic oxides such as silica, alumina, titania, zirconia, and the like; composite oxides; calcium phosphate; hydroxylapatite; yttrium fluoride; ytterbium fluoride; barium titanate; potassium titanate; and the like. Among these, silica, alumina, titania, silica-alumina composite oxide, and silica-zirconia composite oxide are preferably used.

Commercially available products of the inorganic particles (B) include Aerosil R812, R972, and RX-50 (all of which are manufactured by Nippon Aerosil Co., Ltd.).

The refractive index of the inorganic particles (B) is 1.43 to 1.50 and is preferably 1.43 to 1.46.

The difference between the refractive index of the polymer of polymerizable monomer and the inorganic particles (B) is 0.05 or more.

### [Other Components]

The dental restoration composite resin of the present embodiment may further contain a chemical polymerization initiator. The dental restoration composite resin of the present embodiment can be cured at room temperature herewith.

Examples of oxidizing agents include organic peroxides such as diacyl peroxide, peroxy ester, peroxycarbonate, dialkyl peroxide, peroxyketal, ketone peroxide, hydroperoxide, and the like. The above oxidizing agents may be used in combination of two or more.

Examples of diacyl peroxides include benzoyl peroxide, 2,4-dichlorobenzoyl peroxide, m-toluoyl peroxide, lauroyl peroxide, and the like.

Examples of peroxy esters include t-butyl peroxybenzoate, di-t-butyl peroxyisophthalate, t-butyl peroxy-2-ethylhexanoate, and the like.

Examples of peroxycarbonates include t-butyl peroxyisopropyl carbonate and the like.

Examples of dialkyl peroxides include dicumyl peroxide, di-t-butyl peroxide, 2,5-dimethyl-2,5-bis(benzoyl peroxy)hexane, and the like.

Examples of peroxyketals include 1,1-bis(t-butylperoxy)-3,3,5-trimethylcyclohexane and the like.

Examples of ketone peroxides include methyl ethyl ketone peroxide and the like.

Examples of hydroperoxides include t-butyl hydroperoxide and the like.

Examples of reducing agents include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-p-toluidine, N,N-bis(2-hydroxyethyl)-3,5-dimethylaniline, N,N-bis(2-hydroxyethyl)-3,4-dimethylaniline, N,N-bis(2-hydroxyethyl)-4-ethylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, ethyl 4-dimethylaminobenzoate, n-butoxyethyl 4-dimethylaminobenzoate, 2-methacryloyloxyethyl 4-dimethylaminobenzoate, trimethylamine, triethylamine, N-methyldiethanolamine, N-ethyldiethanolamine, N-n-butyldiethyldiethanolamine, N-lauryl diethanolamine, triethanolamine, (2-dimethylamino)ethylmethacrylate, N-methyldiethanolamine dimethacrylate, N-ethyldiethanolamine dimethacrylate, N-ethyldiethanol dimethacrylate, triethanolamine monomethacrylate, triethanolamine dimethacrylate, triethanolamine trimethacrylate, and the like. The above reducing agents may be used in combination of two or more.

The dental restoration composite resin of the present embodiment may further contain a photoinitiator. The dental restoration composite resin of the present embodiment can be cured by being irradiated with visible light, ultraviolet light, or the like.

Examples of photoinitiators used for irradiating visible light include α-diketone, ketal, thioxanthone, and the like.

Examples of α-diketones include camphorquinone, benzyl, 2,3-pentanedione, and the like.

Examples of ketals include benzyl dimethyl ketal, benzyl diethyl ketal, and the like.

Examples of thioxanthones include 2-chlorothioxanthone, 2,4-diethylthioxanthone, and the like.

Photopolymerization initiators used when irradiating visible light are usually used in combination with reducing agents.

Examples of reducing agents include tertiary amines such as Michler ketone, 2-(dimethylamino)ethyl methacrylate, N,N-bis[(meth)acryloyloxyethyl]-N-methylamine, 4-ethyl dimethylaminobenzoate, 4-butyl dimethylaminobenzoate, butoxyethyl 4-dimethylaminobenzoate, N-methyldiethanolamine, 4-dimethylaminobenzophenone, N,N-bis(2-hydroxyethyl)-p-toluidine, dimethylaminophenanthol; aldehydes such as citronellal, lauryl aldehyde, phthaldialdehyde, dimethylaminobenzaldehyde, and terephthalaldehyde; and compounds having a thiol group such as 2-mercaptobenzoxazole, decanthiol, 3-mercaptopropyltrimethoxysilane, 4-mercaptoacetophenone, thiosalicylic acid, and the like.

A photo-initiator, a reducing agent, and an organic peroxide may be used in combination.

Examples of the photo-initiators for irradiating ultraviolet light include benzoin alkyl ether, benzyl dimethyl ketal, acylphosphine oxide, bis-acylphosphine oxide, and the like.

Examples of acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, benzoyl bis (2,6-dimethylphenyl) phosphonate, 2,4,6-trimethylbenzoylethoxyphenyl phosphine oxide, and the like.

Examples of the bis-acylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and the like.

A (bis)acylphosphine oxide may be substituted by a water-soluble substituent.

Alternatively, (bis)acylphosphine oxide may be combined with a reducing agent such as an amine, aldehyde, mercaptan, sulfinate, or the like.

The mass ratio of the polymerizable initiator with respect to the polymerizable monomer is in a range of 0.1% to 10%, preferably in a range of 0.2% to 5%.

The dental restoration composite resin of the present embodiment may further contain a polymerization inhibitor, an ultraviolet absorber, and the like.

Examples of the polymerization inhibitors include 2,6-di-t-butyl-p-cresol, 6-t-butyl-2,4-xylenol, hydroquinone, dibutyl hydroquinone, dibutyl hydroquinone monomethyl ether, 2,6-di-t-butylphenol, 4-methoxyphenol, and the like. The polymerization inhibitors may be used in combination of two or more.

Examples of ultraviolet absorbers include 2-(2H-benzotriazol-2-yl)-4-methylphenol, 2-ethylhexyl 4-dimethylaminobenzoate, 4-aminobenzoic acid, and the like.

### EXAMPLES

Hereinafter, examples of the present invention will be described, but the present invention is not limited to the examples. Note that "part" indicates "part by mass".

### [Manufacture of Inorganic Particles (A1)]

Irregularly-shaped barium glass particles GM27884 NanoFine 180 (manufactured by Schott AG), having a volume-median particle size of 0.18 µm, were surface-treated with 8-methacryloyloxyoctyltrimethoxysilane to obtain inorganic particles (A1) having a volume-median particle size of 0.18 µm.

### [Production of Inorganic Particles (A2)]

Inorganic particles (A2), having a volume-median particle size of 0.18 µm, were prepared in the same manner as the inorganic particles (A1) except that a mixture of 8-methacryloyloxyoctyltrimethoxysilane and 3-methacryloyloxypropyltrimethoxysilane (mass ratio 3:1) was used instead of 8-methacryloyloxyoctyltrimethoxysilane.

### [Production of Inorganic Particles (A3)]

Inorganic particles (A3), having a volume-median particle size of 0.18 µm, were prepared in the same manner as the inorganic particles (A1) except that a mixture of 8-methacryloyloxyoctyltrimethoxysilane and 3-methacryloyloxypropyltrimethoxysilane (mass ratio 1:1) was used instead of 8-methacryloyloxyoctyltrimethoxysilane.

### [Production of Inorganic Particles (A4)]

Inorganic particles (A4), having a volume-median particle size of 0.18 µm, were prepared in the same manner as the inorganic particles (A1) except that a mixture of 8-methacryloyloxyoctyltrimethoxysilane and 3-methacryloyloxypropyltrimethoxysilane (mass ratio 1:3) was used instead of 8-methacryloyloxyoctyltrimethoxysilane.

### [Production of Inorganic Particles (A5)]

Inorganic particles (A5), having a volume-median particle size of 0.18 µm, were prepared in the same manner as the inorganic particles (A1) except that 3-methacryloyloxypropyltrimethoxysilane was used instead of 8-methacryloyloxyoctyltrimethoxysilane.

Table 1 lists the surface treating agents used in producing inorganic particles (A).

**[Table 1]**

| Inorganic particles (A) | Surface treatment agent [% by mass] | |
|---|---|---|
| | 8-methacryloyloxy octyltri methoxysi la ne | 3-methacryloyloxy propyltrimethoxysilane |
| A1 | 100 | 0 |
| A2 | 75 | 25 |
| A3 | 50 | 50 |
| A4 | 25 | 75 |
| A5 | 0 | 100 |

### [Volume-Median Particle Size of Inorganic Particles (A)]

15 mg of the inorganic particles (A) were added to 20 mL of a 0.2% by mass of sodium hexametaphosphate solution. The inorganic particles (A) were dispersed for 30 minutes using an ultrasonic disperser to obtain a dispersion of the inorganic particles (A). The volume-median particle size of the inorganic particles (A) was then measured using a laser diffraction-scattering particle size distribution analyzer LA-950 (manufactured by HORIBA, Ltd).

### [Inorganic Particles (B1)]

As inorganic particles (B1), Aerosil R812 (manufactured by Nippon Aerosil Co., Ltd.) of silica particles, which have been surface-treated with hexamethyldisilazane, having an average primary particle size of 7 nm, were used.

### [Average Primary Particle Size of Inorganic Particles (B1)]

Electron micrographs of 100 inorganic particles (B1) were subjected to image analysis using image analysis software WinROOF (manufactured by MITANI Corporation). Thereafter, an average primary particle size of the inorganic particles (B1) was determined as the volume-median particle size.

### [Preparation of Polymerizable Compositions]

70 parts of 2,2-bis[4-(2-methacryloyloxyethoxy)phenyl]propane (Bis-MEPP), 20 parts of [2,2,4-trimethylhexamethylene bis(2-carbamoyloxyethyl)] dimethacrylate (UDMA), and 10 parts of triethylene glycol dimethacrylate (TEGDMA) were mixed. Appropriate amounts of camphorquinone, ethyl N,N-dimethylaminobenzoate, trimethyldiphenylphosphine oxide, and dibutylhydroxytoluene (BHT) were added to obtain a polymerizable composition.

### [Example 1]

0.1 parts of an α-methylstyrene dimer (MSD) were added to the polymerizable composition, then stirred and homogenized. Consequently. 200 parts of inorganic was then vacuum defoamed to obtain a paste-like dental restoration composite resin.

### [Example 2]

A paste-like dental restoration composite resin was obtained in the same manner as Example 1, except that the inorganic particles (A2) were used instead of the inorganic particles (A1).

### [Example 3]

A paste-like dental restoration composite resin was obtained in the same manner as Example 1, except that the inorganic particles (A3) were used instead of the inorganic particles (A1).

### [Example 4]

A paste-like dental restoration composite resin was obtained in the same manner as Example 1, except that the amount of MSD added was changed to 0.5 parts.

### [Example 5]

A paste-like dental restoration composite resin was obtained in the same manner as in Example 4, except that the inorganic particles (A2) were used instead of the inorganic particles (A1).

### [Example 6]

A paste-like dental restoration composite resin was obtained in the same manner as in Example 4, except that the inorganic particles (A3) were used instead of the inorganic particles (A1).

### [Example 7]

A paste-like dental restoration composite resin was obtained in the same manner as Example 1, except that the amount of MSD added was changed to 1 part.

### [Example 8]

A paste-like dental restoration composite resin was obtained in the same manner as in Example 7, except that the inorganic particles (A2) were used instead of the inorganic particles (A1).

### [Example 9]

A paste-like dental restoration composite resin was obtained in the same manner as in Example 7, except that the inorganic particles (A3) were used instead of the inorganic particles (A1).

### [Example 10]

A paste-like dental restoration composite resin was obtained in the same manner as in Example 7, except that the inorganic particles (A4) were used instead of the inorganic particles (A1).

### [Example 11]

A paste-like dental restoration composite resin was obtained in the same manner as Example 1, except that the amount of MSD added was changed to 5 parts.

### [Example 12]

A paste-like dental restoration composite resin was obtained in the same manner as Example 11, except that the inorganic particles (A2) were used instead of the inorganic particles (A1).

### [Example 13]

A paste-like dental restoration composite resin was obtained in the same manner as Example 11, except that the inorganic particles (A3) were used instead of the inorganic particles (A1).

### [Example 14]

A paste-like dental restoration composite resin was obtained in the same manner as Example 4, except that 1-decanthiol was used instead of MSD.

### [Example 15]

A paste-like dental restoration composite resin was obtained in the same manner as Example 7, except that 1-decanthiol was used instead of MSD.

### [Example 16]

A paste-like dental restoration composite resin was obtained in the same manner as Example 9, except that 1-decanthiol was used instead of MSD.

### [Example 17]

A paste-like dental restoration composite resin was obtained in the same manner as Example 4, except that γ-terpinene was used instead of MSD.

### [Example 18]

A paste-like dental restoration composite resin was obtained in the same manner as Example 7, except that γ-terpinene was used instead of MSD.

### [Example 19]

A paste-like dental restoration composite resin was obtained in the same manner as Example 9, except that γ-terpinene was used instead of MSD.

### [Example 20]

A paste-like dental restoration composite resin was obtained in the same manner as Example 4, except that cumyl dithiobenzoate (DACE) was used instead of MSD.

### [Example 21]

A paste-like dental restoration composite resin was obtained in the same manner as Example 7, except that cumyl dithiobenzoate (DACE) was used instead of MSD.

### [Example 22]

A paste-like dental restoration composite resin was obtained in the same manner as Example 9, except that cumyl dithiobenzoate (DACE) was used instead of MSD.

### [Example 23]

A paste-like dental restoration composite resin was obtained in the same manner as Example 1, except that the inorganic particles (A5) were used instead of the inorganic particles (A1).

### [Example 24]

A paste-like dental restoration composite resin was obtained in the same manner as Example 11, except that the inorganic particles (A5) were used instead of the inorganic particles (A1).

### [Comparative Example 1]

A paste-like dental restoration composite resin was obtained in the same manner as Example 1, except that MSD was not added.

Next, the bending strength of the cured product, the shrinkage stress upon curing, and handleability of the dental restoration composite resin were evaluated.

### [Bending Strength of Cured Product]

After filling a dental restoration composite resin in a stainless-steel mold of 2 mm × 2 mm × 25 mm, the flowable composite resin was brought into press contact with slide glasses on the upper side and the lower side. Next, the flowable composite resin was cured by irradiating the upper surface and the lower surface at nine points on each surface with visible light for 10 seconds per point, using a G-Light Prima-II (manufactured by GC Corporation). Then, after being extracted from the mold, the cured product was stored in distilled water at 37°C for 24 hours to obtain a test piece. At this point, five test pieces were prepared. Next, the bending strength of the five test pieces was measured using a universal testing machine AG-IS (manufactured by Shimadzu Corporation) with the support span being 20 mm and the crosshead speed being 1 mm/min. Thereafter, the average value of the five test pieces was calculated and determined as bending strength.

### [Shrinkage Stress upon Curing]

Autograph EZ-S 500N (manufactured by Shimadzu Corporation) was used to measure the shrinkage stress of the dental restoration composite resin upon curing. Specifically, Metalprimer Z (manufactured by GC Corporation) and G-Premio BOND (manufactured by GC Corporation) were sequentially applied to the surface of an aluminum tube and a stainless rod with an inner diameter of 5 mm. Visible light was then irradiated to the surfaces of the aluminum tube and stainless rod for 10 seconds using a G-light prima II (manufactured by GC Corporation). Sequentially, the aluminum tube and stainless rod were then attached to the autograph and then filled with the dental restoration composite resin to a depth of 2 mm in the aluminum tube. The crosshead speed was then set to 0 mm/min. Visible light was irradiated to the dental restoration composite resin for 10 seconds using a G-light prima II (manufactured by GC Corporation) to cure the dental restoration composite resin. At this time, the change in stress up to 10 minutes was measured, and the maximum point was determined as the shrinkage stress upon curing.

### [Handleability]

Using the above-described syringe, plunger, and needle chip, 0.03 g of a flowable composite resin was extruded onto white mixing paper. At this point, the handleability was evaluated.

The handleability was determined based on the following criteria.
A: The viscosity of the dental restoration composite resin is adequate, extrusion from the syringe is very easy, and the shape of the flowable composite resin is more easily built up and corrected.
B: The viscosity of the dental restoration composite resin is adequate, extrusion from the syringe is easy, and the shape of the flowable composite resin is easily built up and corrected.
C: The viscosity of the dental restoration composite resin is high, extrusion from the syringe is possible, and the shape of the flowable composite resin is possibly corrected.
D: The viscosity of the dental restoration composite resin is high, extrusion from the syringe is difficult, and the shape of the flowable composite resin is hardly corrected.

Table 2 indicates the evaluation results of the bending strength of the cured product, the shrinkage stress upon curing, and handleability of the dental restoration composite resin.

**[Table 2]**

| | Chain transfer agent | | Inorganic particles (A) | Bending strength of cured product [MPa] | Shrinkage stress upon curing [MPa] | Handle ability |
|---|---|---|---|---|---|---|
| | Type | The molar ratio with respect to polymerizable monomer [%] | | | | |
| Example 1 | MSD | 0.1 | A1 | 150 | 0.40 | A |
| Example 2 | MSD | 0.1 | A2 | 145 | 0.43 | A |
| Example 3 | MSD | 0.1 | A3 | 142 | 0.41 | B |
| Example 4 | MSD | 0.5 | A1 | 145 | 0.40 | A |
| Example 5 | MSD | 0.5 | A2 | 143 | 0.40 | A |
| Example 6 | MSD | 0.5 | A3 | 145 | 0.38 | B |
| Example 7 | MSD | 1 | A1 | 159 | 0.35 | A |
| Example 8 | MSD | 1 | A2 | 160 | 0.33 | A |
| Example 9 | MSD | 1 | A3 | 132 | 0.45 | B |
| Example 10 | MSD | 1 | A4 | 144 | 0.36 | C |
| Example 11 | MSD | 5 | A1 | 120 | 0.20 | A |
| Example 12 | MSD | 5 | A2 | 112 | 0.19 | A |
| Example 13 | MSD | 5 | A3 | 115 | 0.23 | B |
| Example 14 | 1-decanthiol | 0.5 | A1 | 156 | 0.53 | A |
| Example 15 | 1-decanthiol | 1 | A1 | 151 | 0.51 | A |
| Example 16 | 1-decanthiol | 1 | A3 | 144 | 0.50 | B |
| Example 17 | γ-terpinene | 0.5 | A1 | 149 | 0.49 | A |
| Example 18 | γ-terpinene | 1 | A1 | 148 | 0.49 | A |
| Example 19 | γ-terpinene | 1 | A3 | 141 | 0.45 | B |
| Example 20 | DACE | 0.5 | A1 | 153 | 0.48 | A |
| Example 21 | DACE | 1 | A1 | 148 | 0.19 | A |
| Example 22 | DACE | 1 | A3 | 140 | 0.17 | B |
| Example 23 | MSD | 1 | A5 | 145 | 0.49 | D |
| Example 24 | MSD | 5 | A5 | 98 | 0.32 | D |
| Comparative Example 1 | - | 0 | A1 | 155 | 0.55 | A |

From Table 2, it can be seen that the dental restoration composite resins of Examples 1 to 24 have low shrinkage stress upon curing and high bending strength of the cured products.

In contrast, the dental restoration composite resin of Comparative Example 1 did not contain the chain transfer agent and thus had high shrinkage stress upon curing.

## Claims

1. A dental restoration composite resin comprising:
a polymerizable monomer;
a chain transfer agent; and
inorganic particles,
wherein the inorganic particles contain inorganic particles (A) having a volume-median particle size of 0.1 µm or more and 0.9 µm or less, the inorganic particles (A) being surface-treated with compounds represented by the following general formula (1), the following general formula (2), or both, 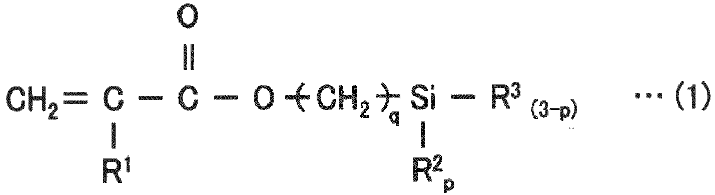
wherein R¹ is a hydrogen atom or a methyl group, R² is a hydrolyzable group, R³ is a hydrocarbon group of 1 to 6 carbon atoms, p is 2 or 3, and q is an integer of 8 to 13, and 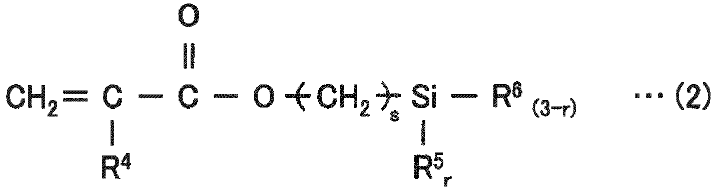
wherein R⁴ is a hydrogen atom or a methyl group, R⁵ is a hydrolyzable group, R⁶ is a hydrocarbon group of 1 to 5 carbons, r is 2 or 3, and s is an integer of 1 to 7.

2. The dental restoration composite resin according to claim 1, wherein the chain transfer agent is an α-methyl styrene dimer, 1-decanthiol, γ-terpinene, or cumyl dithiobenzoate.

3. The dental restoration composite resin according to claim 1 or 2, wherein a mass ratio of the chain transfer agent with respect to the polymerizable monomer is 0.1% or more and 5% or less.

4. The dental restoration composite resin according to any one of claims 1 to 3, wherein a mass ratio of the compound represented by the general formula (1) with respect to the compound represented by the general formula (2) is 1 or more.

5. The dental restoration composite resin according to any one of claims 1 to 4, further comprising inorganic particles (B) in the inorganic particles,
wherein an average primary particle size of the inorganic particles (B) is 5 nm or more and 50 nm or less,
wherein the inorganic particles (B) have groups represented by the following general formula (3), the following general (4), or both at the surfaces of inorganic particles, 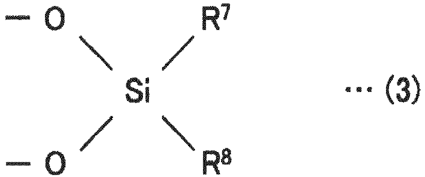 wherein R⁷ and R⁸ are independently a methyl group or an ethyl group, and 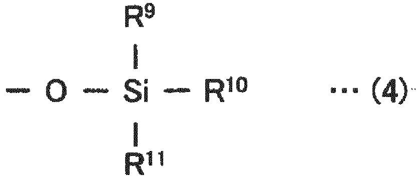 wherein R⁹, R¹⁰, and R₁₁ are independently a methyl group or an ethyl group.
